# EUROPEAN PATENT APPLICATION

(11) **EP 3 959 995 A1**
(43) Date of publication of application: **02.03.2022**
(21) Application number: 21382722.3
(22) Date of filing: 30.07.2021
(51) Int. Cl.: A23L 27/12, A61K 31/7048, A61K 36/752, A61P 39/06

(54) **POWDERED FOOD COMPOSITION COMPRISING ERIOCITRIN**

(30) Priority: 30.07.2020 ES 202031703 U
(71) Applicant: Euromed, S.A., 08100 Mollet Del Vallès (Barcelona) (ES)
(72) Inventor: ROIG ALMIRALL, FRANCISCO JAVIER, 08021 BARCELONA (ES); VILLAR GONZALEZ, AGUSTIN, 08291 RIPOLLET (BARCELONA) (ES); GUERRERO MARTINEZ, JUAN RAMON, 30152 ALJUCER (MURCIA) (ES)
(74) Representative: Durán-Corretjer, S.L.P.

(57) **Abstract**

The present invention refers to a powdered food composition, obtained from lemon characterized in that it comprises between 1% and 30% (w/w) of eriocitrin.

## Description

The present invention relates to a powdered food composition comprising between 1% and 30% (w/w) of eriocitrin, the most abundant flavanone in lemon, to be used as a food supplement due to its beneficial properties. The present invention also relates to a powdered food composition for use in inflammation, cancer, neurodegenerative diseases, diabetes, cardiovascular diseases, vascular diseases and cutaneous diseases.

Flavanones are the most abundant flavonoids in most citrus species. The structure of flavanones differs chemically from flavone in the saturation of the bond between carbons 2 and 3, and in the disappearance, due to this fact, of the conjugation between said double bond and the carbonyl group, conjugated in turn with the 2-phenyl group ring B and with ring A. Its basic model is therefore 2-phenylbenzopyran-4-one.

The main flavanones of the genus Citrus are hesperetin, naringenin, eriodictyol and isosakuranetin, which are mostly found in glycosylated form at the hydroxyl located at the C-7 carbon. This glycosylation can be of two types depending on the structure: (a) β-neohesperidoside (2-OaL-rhamnopyranosyl-β-D-glucopyranose) and (b) β-rutinoside (6-OaL-rhamnopyranosyl-β-D-glucopyranose). Within the rutinosides, the most abundant flavanones in citrus fruits are hesperidin, narirutin and eriocitrin.

The most abundant flavanone in citrus extracts like orange is hesperidin. However, there is another relevant and representative flavanone in lemon, known as eriocitrin. Eriocitrin has an important antioxidant capacity and is much more soluble in water than hesperidin thanks to the presence of two hydroxyl groups (-OH) in the B ring of the flavonoid structure. It is reported that water solubility of eriocitrin is about 40 mg/ml while water solubility of hesperidin is just 4.9 µg/ml. The eriocitrin content in commercial citrus extracts is very low due to inappropriate processes and/or raw materials used in industry. For this reason, it is not uncommon to find eriocitrin when the actual compound is hesperidin, or to sell as a lemon extract what is actually orange extract with hesperidin. Low solubility in water makes purification process of hesperidin much easier than the process of eriocitrin, obtaining hesperidin at high purities with relatively simple process. That is why hesperidin is the main flavonone from citrus found in the market. There are numerous scientific studies related to hesperidin thanks to its common incorporation in the diet and the ease of obtaining it. However, there are few scientific studies related to eriocitrin.

The science developed in this regard supports the numerous health benefits of orange consumption, thanks to its high hesperidin content. Among all these advantages, its anti-inflammatory, lipid-lowering, hypotensive and venotonic activity stands out. Its use against osteoporosis has also been described. The benefits of hesperidin are associated with the metabolites that circulate in the body after ingestion of the extract, mainly hesperetin conjugates, such as glucuronides, sulfates, among others. However, human studies on the bioavailability and metabolism of eriocitrin are much less abundant.

Epidemiological studies and meta-analyses have associated the intake of flavonoid rich citrus fruit consumption with lower stroke risk (Cassidy, A. et al., Dietary flavonoids and risk of stroke in women. stroke 2012, 43, 946-951) and the incidence of some types of cancer (McCullough, M.L. et al., A prospective study of diet and stomach cancer mortality in United States men and women. Cancer Epidemiol. Biomark. Prev. 2001, 10, 1201; Gates, M.A. et al., A prospective study of dietary flavonoid intake and incidence of epithelial ovarian cancer. Int. J. Cancer 2007, 121, 2225-2232).

Eriocitrin as a glycosylated compound must be transformed into different active metabolites to exert its beneficial activities for health. The main known active metabolites are eriodictyol and homoeriodictyol. Eriocitrin is a glycosylated compound that needs transformation to be absorbed in the bowel and reach to the bloodstream. Compounds without sugar (aglycones) reach to the blood and then to other tissues exerting its potential health benefits.

Eriodictyol seems to be associated with its ability to modulate a number of cell-signaling cascades. Several studies have indicated that eriodictyol exerts multiple therapeutic effects including antioxidant (Chobot et al., Pro- and antioxidant activity of three selected flavan type flavonoids: catechin, eriodictyol and taxifolin. Int J Mol Sci. 2016, 17(12), 1986), anti-inflammation (Zhu et al., Eriodictyol, a plant flavonoid, attenuates LPS-induced acute lung injury through its antioxidative and anti-inflammatory activity. Exp Ther Med. 2015, 10:2259-2266), anti-cancer (Zhang et al., Eriodictyol exerts potent anticancer activity against A549 human lung cancer cell line by inducing mitochondrial-mediated apoptosis, G2/M cell cycle arrest and inhibition of m-TOR/PI3K/Akt signalling pathway. Arch Med Sci. 2020, 16:446-452), neuroprotection (Habtemariam, The Nrf2/HO-1 axis as targets for flavanones: neuroprotection by pinocembrin, naringenin, and eriodictyol. Oxid Med Cell Longev. 2019, 4724920), cardioprotection (Li et al., Eriodictyol attenuates myocardial ischemia-reperfusion injury through the activation of JAK2. Front Pharmacol. 2018, 9:33), anti-diabetic (Zhang et al., Effect of eriodictyol on glucose uptake and insulin resistance in vitro. J Agric Food Chem. 2012, 60:7652-7658), anti-obesity (Kwon and Choi, Dietary eriodictyol alleviates adiposity, hepatic steatosis, insulin resistance, and inflammation in diet-induced obese mice. Int J Mol Sci. 2019, 20(5), 1227), hepatoprotection (Xie et al., Eriodictyol attenuates arsenic trioxide-induced liver injury by activation of Nrf2. Oncotarget. 2017, 8:68668-68674), and miscellaneousness (Arakaki et al., Novel effects of rooibos extract on tear and saliva secretion mediated by the muscarinic acetylcholine receptor 3 in mice. J Oral Biosci. 2019, 61:179-182). Eriodictyol regulates the expression of Heme-oxygenase-1 (HO1) enzyme associated with the ERK/Nrf2/ARE signaling pathway, potentially reducing H2O2-induced damage at the endothelial level. This mechanism will allow an eriodictyol (eriocitrin-derived metabolite) to have a potential therapeutic application as a protector against diseases related to oxidative stress, including cardiovascular diseases (Lee et al., Eriodictyol Protects Endothelial Cells against Oxidative Stress-Induced Cell Death through Modulating ERK/Nrf2/ARE-Dependent Heme Oxygenase-1 Expression. Int. J. Mol. Sci. 2015, 16(7), 14526-14539).

Other pre-clinical studies have been able to show that with low concentrations of homoeriodictyol (another important metabolite derived from eriocitrin), therapeutic effects such as inhibition of platelet aggregation, increased coronary flow, and support for blood vessel dilation were observed. This together with the antioxidant capacity will allow to prevent the incidences of vascular diseases (Shen et al., Homoeriodictyol protects human endothelial cells against oxidative insults through activation of Nrf2 and inhibition of mitochondrial dysfunction. Vascular Pharmacology. 2017).

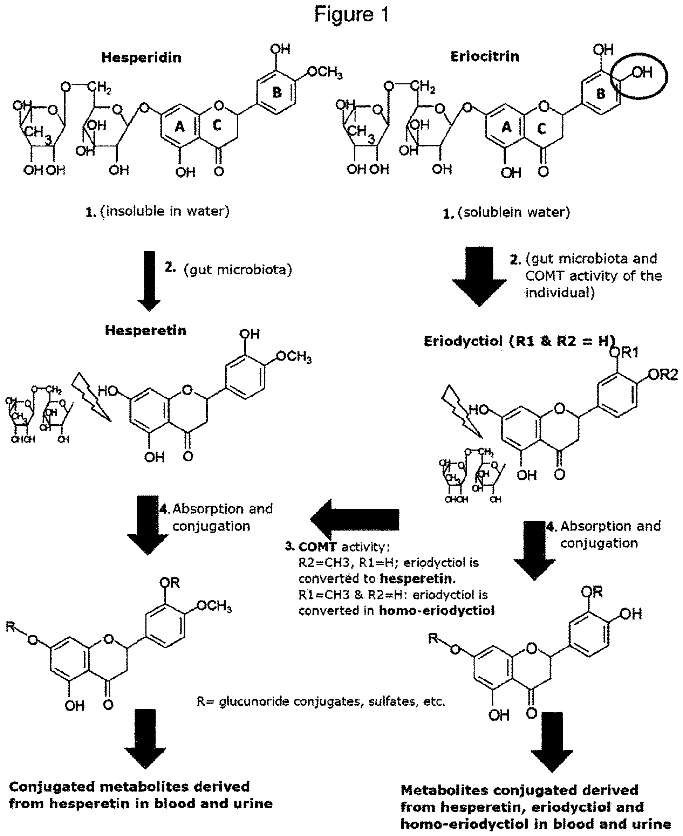

Citrus flavonoids are already known for their multiple health benefits, including their molecular structures that will determine how powerful the final effect will be (Tripoli et al., Citrus flavonoids: Molecular structure, biological activity and nutritional properties: A review. Food Chemistry. 2007, 104, 466-479). The common factor of this group of flavonoids is the low solubility in water that almost all have, and this is an important point to consider regarding bioavailability (Ávila-Gálvez et al., New Insights into the Metabolism of the Flavanones Eriocitrin and Hesperidin: A Comparative Human Pharmacokinetic Study. Antioxidants. 2021, 10(3):435). Hesperidin, despite being a practically insoluble molecule (5 micrograms/ml) in water and not very bioavailable, is used only to support the venous system, edema, varicose veins, among others (Man et al., Benefits of Hesperidin for Cutaneous Functions. Evidence-Based Complementary and Alternative Medicine 2019, Article ID 2676307). The same happens with diosmin, a semi-synthetic compound derived from hesperidin with a very low solubility (20 micrograms/ml) in water, which is known and probably the most widely used for the same purpose as hesperidin. Relationship between low solubility in water and a low bioavailavility is related to the intestinal microbiota. It has been mentioned previously that to reach the bloodstream through the cells of the intestine, it is necessary to remove the sugar from glycosylated flavanone. This deglycosylation is carried out by the intestinal microbiota and only water-soluble molecules are capable of accessing the interior of these microorganisms. The amounts of glycosylated flavonoids (natural form of the compound) that reach the interior of the intestinal microbiota is proportional to their solubility (see Figure 1).

The pioneering study in humans developed by Ávila-Gálvez et al. in 2021, which compares the bioavailability of hesperidin versus eriocitrin, clearly shows that, once in human cells, both eriodictyol and hesperetin produce different metabolites that can be detected in blood and urine (Miyake et al., Identification and Antioxidant Activity of Flavonoid Metabolites in Plasma and Urine of Eriocitrin-Treated Rats. J. Agric. Food Chem. 2000, 48, 3217-3224). A novelty found in the study is that not only metabolites of this flavanone are found in the blood and urine of volunteers who take eriocitrin, but also metabolites characteristic of volunteers who have ingested only hesperidin. This does not happen the other way around, eriocitrin metabolites in blood and urine were not found in volunteers who have ingested only hesperidin. This chemical reaction, in the body, plus a high solubility (4000 micrograms/ml) could present an advantage when it comes to exerting the common health benefits for all citrus flavanones, since the amount of final active metabolites, which can form a molecule of eriocitrin, they will be much superior compared to, for example, those that can potentially create hesperidin.

On the market, there are drugs with hesperidin and/or its semisynthetic analog, diosmin. The indication for its sale is as a venotonic agent, that is, to improve capillary permeability. The alteration of patency causes the appearance of fluid retention, varicose veins, hemorrhoids and venous insufficiency in the lower extremities. The drug, marketed under the trade name Daflon^{®}, has long been widely sold to a wide spectrum of the population, including pregnant women. Currently, the European Food Safety Authority (EFSA) has rejected the indication associated with the consumption of hesperidin and analogues for the "maintenance of normal permeability of capillaries and veins" (EFSA Journal 2014; 12 (1): 3511). This prohibition could be due to the great variability of the results obtained in the scientific field. This variability could be due to the disparity in the response to the intake of polyphenols such as hesperidin flavanone, which could be the result of hesperidin's insolubility. Large multinational pharmaceutical companies are developing studies to solve the problem of the insolubility of this extract. To overcome this limitation, eriocitrin can play a relevant role.

The insolubility of hesperidin is extremely important from two points of view: organoleptic and functional. Hesperidin forms a suspended cloud that precipitates to the bottom in freshly squeezed orange juices. In juices, industrial drinks and freshly squeezed drinks, hesperidin is found in an insoluble form. The agitation of the juice promotes the suspension but not the disolution of the insoluble hesperidin. The intestinal microbiota is essential for the metabolization of hesperidin, the absorption of metabolites and their consequent effect on the body.

The intestinal microbiota hydrolyzes the glycoside group, known as rutinose, giving rise to the hesperetin molecule. This hydrolysis is essential for the absorption of this molecule (Vallejo et al., Concentration and Solubility of Flavanones in Orange Beverages Affect Their Bioavailability in Humans. J. Agric. Food Chem. 2010, 58, 6516-6524). If hesperidin is insoluble, it is difficult for the microbiota responsible for breaking the sugar group to access it, therefore little hesperetin will be formed and bioavailability will decrease. On the contrary, if the solubility improves, the bioavailability will increase (Tomás-Navarro et al., Chapter 40 - Bioavailability and Metabolism of Citrus Fruit Beverage Flavanones in Humans. Polyphenols in Human Health and Disease. 2014, 1, 537-551). Eriocitrin has been studied much less due to its difficult obtaining in conventional extraction processes and/or because the raw material used is not appropriate. However, the lipid-lowering and antioxidant effect of eriocitrin, capable of activating mitochondrial biogenesis and reducing nonalcoholic fatty liver disease, has been described. However, due to their similarity it is likely that their effects are similar to or better than those described for hesperidin.

A lemon extract high in eriocitrin is much more soluble in water than an extract rich in hesperidin. The solubility can improve its use in functional foods, since it will facilitate the bioavailability of its bioactive metabolites. Therefore, after the release of the sugar, the resulting molecule, eriodictyol, can be converted into hesperetin thanks to the action of the enzyme catechol-O-methyl-transferase (COMT) in the intestine and liver, which introduces methyl groups (-CH3) in molecules in the 'ortho' position, since this is the case of eriodictyol in its B ring.

The inventors of the present invention have found that eriocitrin, being soluble, is much more accessible to the colonic microbiota than hesperidin in breaking down the rutin residue. Therefore, eriocitrin is an ideal way to obtain the same final active metabolite (hesperetin) but generating it *in situ,* in the intestine. Thus, the lemon extract increases the effects described for hesperidin and, in addition, the possible presence of eriodictyol metabolites with the two -OH groups in the 'ortho' position, presumably exerts a high antioxidant activity.

Compositions containing eriocitrin are known. However, said compositions have a high content of eriocitrin, around 70% (w/w), such as that marketed under the trade name Eriomin^{®}, which means that it has been obtained using purification methods of said flavanone. In these methods other beneficial flavonoids present in lemon are also eliminated, such as apigenin 6,8-di-C-Glu (Vicenin-2), chrysoeriol 6,8-di-C-Glu and/or limocitrin HMG-Glu, among others. Therefore, there is a need to obtain compositions containing eriocitrin obtained from lemon with a suitable concentration, which maintains its properties and is more similar to how it is found in its natural state.

The inventors of the present invention have developed a process for obtaining a powdered food composition from lemon, which comprises an extraction stage with a solvent and a tangential flow filtration stage, with which it is possible to obtain eriocitrin with adequate quality characteristics, maintaining its properties, and with a greater similarity to how this flavanone is found in its natural environment. Preferably, a method to obtain the lemon extract based in tangential flow filtration with a membrane size that ranges between 100 Da and 100,000 Da.

In one aspect, the inventors of the present invention have developed a powdered food composition comprising between 1% and 30% (w/w) eriocitrin.

In addition, the composition can comprise other flavonoids. Preferably, said flavonoids are apigenin 6,8-di-C-Glu (Vicenin-2), chrysoeriol 6,8-di-C-Glu and/or limocytrin HMG-Glu. Preferably, said flavonoids are in a concentration between 0.1 and 3% (w/w).

Preferably, the eriocitrin is extracted from a lemon extract. Most preferably, the eriocitrin is extracted from the lemon fruit. Even more preferably, the eriocitrin is extracted from the fruit of Citrus limon (L.) Osbeck.

The present invention also discloses a method for obtaining a powder composition in which the eriocitrin is obtained by a process comprising a solvent extraction step and a tangential flow filtration step.

Preferably, the size of the membrane in tangential flow filtration is between 100 Da and 100,000 Da.

Finally, the present invention discloses a powdered food composition for use in the treatment of a disease selected from the group consisting of inflammation, cancer, neurodegenerative diseases, diabetes, cardiovascular diseases, vascular diseases and cutaneous diseases in which said composition is administered in a dose of eriocitrin between 5 and 35 mg per day.

Preferably, the eriocitrin is administered in a dose between 7 and 30 mg per day.

Some embodiments of the present invention are described in more detail below by reference to the accompanying schematic figures in which:

### BRIEF DESCRIPTION OF THE FIGURES

Figure 1 represents a diagram of the comparative metabolism of hesperidin and eriocitrin.
Figure 2 represents chromatograms obtained at 340 nm of the orange extract and the lemon extract, respectively. The number of each chromatographic peak refers to Table 1.

As indicated in Figure 1, eriocitrin, being soluble, allows the intestinal microbiota to access it and break down the rutin residue. Once this happens, the resulting molecule, eriodictyol, is able to enter the cells of the intestine where it is converted, at least in part, to hesperetin by the action of the COMT enzyme (point 3, figure 1). The hesperetin is then conjugated and circulates through the body. Therefore, eriocitrin is an ideal way to obtain the same final active metabolite, hesperetin, but generating it in situ, in the intestine.

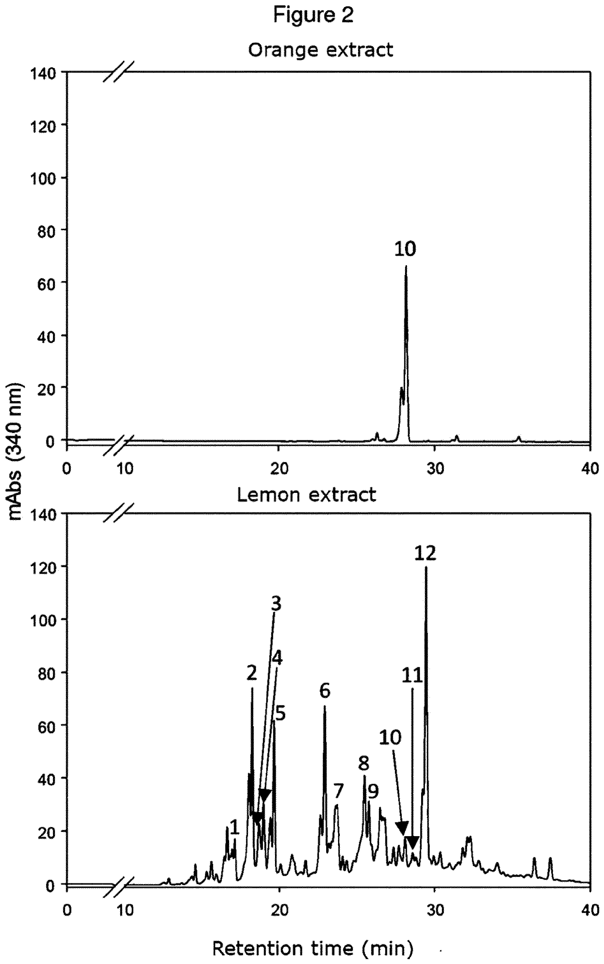

As can be seen in Figure 2, the 'orange extract' consisted exclusively of hesperidin (compound 10, table 1) while the lemon extract presented a much richer profile of compounds. In addition to eriocitrin (compound 6, table 1) as the major compound, two other flavanones were identified in the lemon extract: eriodictyol-glucoside-rhamnoside-glucoside (compound 3, table 1) and naringin (compound 9, Table 1).

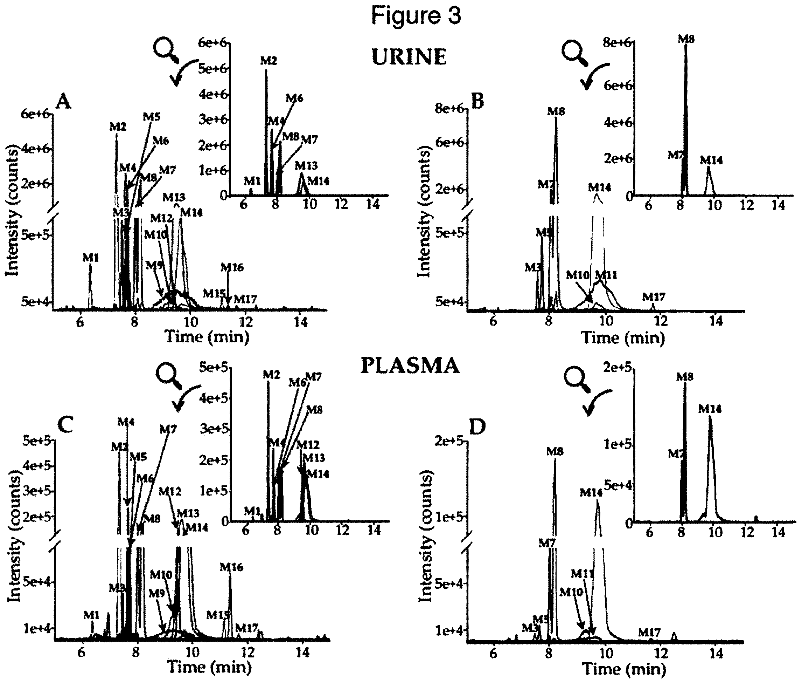

Figure 3 represents a extracted ion chromatograms (EICs) of urine and plasma metabolites after consuming (A,C) lemon or (B,D) orange extract. Numbers designate the metabolites according to Table 3.

Unless defined otherwise, all technical and scientific terms used herein have the same meaning as commonly understood by one of ordinary skill in the art pertinent to the methods and compositions described. As used herein, the following terms and phrases have the meanings ascribed to them unless specified otherwise.

The terms "a," "an," and "the" include plural referents, unless the context clearly indicates otherwise.

Throughout this specification, unless the context requires otherwise, the word "comprise", or variations such as "comprises" or "comprising", will be understood to imply the inclusion of a stated element or integer or group of elements or integers but not the exclusion of any other element or integer or group of elements or integers.

Unless otherwise defined, all technical and scientific terms used herein have the same meaning as commonly understood by one of ordinary skill in the art to which this invention pertains. Exemplary methods and materials are described below, although methods and materials similar or equivalent to those described herein can also be used and will be apparent to those of skill in the art. All publications and other references mentioned herein are incorporated by reference in their entirety. In case of conflict, the present specification, including definitions, will control. The materials, methods, and examples are illustrative only and not intended to be limiting.

Each embodiment in this specification is to be applied mutatis mutandis to every other embodiment unless expressly stated otherwise.

The following terms, unless otherwise indicated, shall be understood to have the following meanings:
The term "treatment" or "treating" means any treatment of a disease or disorder in a subject, such as a mammal, including: preventing or protecting against the disease or disorder, that is, causing the clinical symptoms not to develop; inhibiting the disease or disorder, that is, arresting or suppressing the development of clinical symptoms; and/or relieving the disease or disorder that is, causing the regression of clinical symptoms. In some embodiments, the term treatment is used for the treatment of inflammation, cancer, neurodegenerative diseases, diabetes, cardiovascular diseases, vascular diseases, cutaneous diseases.

It will be understood by those skilled in the art that in human medicine, it is not always possible to distinguish between "preventing" and "suppressing" since the ultimate inductive event or events may be unknown, latent, or the patient is not ascertained until well after the occurrence of the event or events. Therefore, as used herein the term "prophylaxis" is intended as an element of "treatment" to encompass both "preventing" and "suppressing" as defined herein.

Hereinafter, the present invention is described in more detail with reference to illustrative examples, which does not constitute a limitation of the present invention.

### EXAMPLES

Example 1: Obtaining a composition of the present invention with a 10 times higher eriocitrin content.

To obtain a composition of the present invention with eriocitrin, 10 kg of lemon ground was extracted with water at 50°C for 4 hours. Subsequently, by centrifugation, the liquid was separated from the undissolved solid. The extract contained the compound of interest, eriocitrin, a flavonoid abundant in citrus.

The micelle with a dry residue of 5% (w/w) was subjected to a purification process by cascade tangential filtration. For them, 3 chained stages were carried out:
1. The micelle was filtered through a 100,000 Da membrane, passing 99% (w/w) of the eriocitrin to the permeate and obtaining 3% (w/w) of dry residue.
2. The permeate from the first stage was filtered through a 10,000 Da membrane passing the compound of interest to the permeate at 99% (w/w) and obtaining 1% (w/w) of solids.
3. The permeate from stage 2 was filtered through a 1,000 Da membrane passing the eriocitrin at 98% (w/w) to the permeate and obtaining 0.5% (w/w) of solids.

Overall, the three stages went from a liquid extract of 5% (w/w) of solids to one of 0.5% (w/w) of solids, maintaining the same amount of eriocitrin. Therefore, the eriocitrin was concentrated 10 times.

Example 2: Obtaining a composition of the present invention with an eriocitrin content 18 times higher.

To obtain a composition of the present invention with eriocitrin, 10 kg of lemon ground was extracted with water at 50°C for 4 hours. Subsequently, by centrifugation, the liquid was separated from the undissolved solid. The extract contained the compound of interest, eriocitrin.

The micelle with a dry residue of 5% (w/w) was subjected to a purification process by cascade tangential filtration. For them, 3 chained stages were carried out:
1. The micelle was filtered through a 100,000 Da membrane, passing 99% (w/w) of the eriocitrin to the permeate and obtaining 3% (w/w) of dry residue.
2. The permeate from the first stage was adjusted to a basic pH by adding an alkaline substance (sodium hydroxide) and the solution was filtered through a 10,000 Da membrane passing the compound of interest to the permeate by 99% (w/w). The pH caused a greater retention of solids with which a 0.5% (w/w) of solids was finally obtained in the permeate.
3. The permeate from step 2 was filtered through a 1000 Da membrane passing the eriocitrin at 98% (w/w) to the permeate and obtaining 0.25% (w/w) of solids.

Overall, the three stages went from a liquid extract of 5% (w/w) of solids to one of 0.25% (w/w) of solids, maintaining the same amount of eriocitrin. Therefore, the eriocitrin was concentrated a total of 18 times.

Example 3: Characterization of the compounds identified in the lemon and orange extracts by HPLC-UV-MS/MS.

For the identification and quantification of the compounds present in the extracts, the supernatants and precipitates were analyzed by HPLC-UV-MS/MS. For the lemon extract, a single extraction was sufficient since no precipitate appeared after centrifugation. In the case of the orange extract, the precipitate formed was dissolved in 5 mL of DMSO and after centrifugation, no precipitate formed again.

The compounds identified in both extracts are listed in Table 1, ordered by their retention time (TR), their identified mass information (negative ion, [MH]-), their fragmentation profile (MS/MS) and their Maximum absorbance in UV (λmax).

**Table 1. Compounds identified in lemon and orange extracts through HPLC-UV-MS/MS.**

| **Num.** | **Compounds** | **TR** | **[M-H]⁻** | **MS/MS** | **λₘₐₓ** |
|---|---|---|---|---|---|
| **1** | Ferulic Acid O-Glu | 16.94 | 355 | 193/160/134 | 294/328 |
| **2** | Apigenin 6,8-di-C-Glu (Vicenin-2) | 18.13 | 593 | 503/473/383/353 | 270/332 |
| **3** | Eriodictyol-Glu-Ramn-Glu | 18.70 | 757 | 595/449/287 | 282/330 |
| **4** | Diosmetin 6,8-di-*C*-Glu | 19.00 | 623 | 605/533/503/383 | 270/346 |
| **5** | Chrysoeryol 6,8-di-*C*-Glu | 19.65 | 623 | 533/503/383 | 270/346 |
| **6** | Eriocitrin | 22.90 | 595 | 459/329/287 | 284/334 |
| **7** | Apigenin 8-*C*-xylanopyranosyl-Glu | 23.39 | 563 | 413/341/293 | 268/332 |
| **8** | Diosmetin 8-*C*-Glu | 24.88 | 461 | 371/341 | 268/334 |
| **9** | Naringenin 7-*O*-rutinoside (Naringin) | 25.02 | 579 | 271 | 278/330 |
| **10** | Hesperidin^{a} | 28.10 | 609 | 301 | 284/336 |
| **11** | Diosmetin 7-*O*-rutinoside (Diosmin) | 28.40 | 607 | 299/284 | 270/334 |
| **12** | Limocitrin HMG-Glu | 29.43 | 651 | 549/507/345 | 274/350 |

| | | | | | |
|---|---|---|---|---|---|
| ^{a}Only compound present in orange extract. Glu, glucoside. Ramn, ramnoside. HMG, 3-hydroxy-3-methylglutaryl | | | | | |

The chromatograms of the supernatants analyzed by HPLC-UV-MS/MS of each of the extracts are shown in Figure 2, where all the compounds identified in each extract can be seen numbered. The number of each chromatographic peak refers to Table 1.

As can be seen in Figure 2, the 'orange extract' consisted exclusively of hesperidin (compound 10, Table 1) while the lemon extract presented a much richer profile of compounds. In addition to eriocitrin (compound 6, table 1) as the major compound, two other flavanones were identified in the lemon extract: eriodictyol-glucoside-rhamnoside-glucoside (compound 3, table 1) and naringin (compound 9, table 1).

Other phenolic compounds from different families were also identified. For example, the ferulic acid glycoside (compound 1, table 1) was identified which is a hydroxycinnamic acid, and different flavones were also identified, which were different C-glycoside derivatives of: apigenin (compound 2, table 1), diosmetin (compound 4, table 1), and chrysoeriol (compound 5, table 1). The C-glycoside derivatives showed a characteristic fragmentation pattern [(M-H) -90] and [(M-H) -120]. A mono-glucoside (compound 8, table 1) and its rutinoside derivative, diosmin (compound 11, table 1) were also identified from diosmetin. Furthermore, another identified apigenin derivative was apigenin 8-C-xylanpyranosyl-glucoside (compound 7, table 1). Like flavanones, flavones have an absorbance maximum around 340 nm, but with a very different UV spectrum, which makes the compounds of these two families easily differentiable.

Finally, a flavonol, limocytrin-3-hydroxy-3-methyl-glutaryl glucoside (compound 12, table 1) was identified, observing its absorption maximum around 360 nm characteristic of this family of phenolics.

Regarding quantification, it was done for those major compounds of both extracts (Table 2). The main flavanones detected in the lemon and orange extracts were eriocitrin (83.3 ± 5.6 mg / g extract) and hesperidin (133.7 ± 15.9 mg / g extract) respectively. Both compounds were quantified with their own standards.

**Table 2. Compounds quantified in the orange and lemon extracts.**

| Num | Compounds | mg/g extracto ± DE |
|---|---|---|
| 2 | Apigenin 6,8-di-*C*-Glu (Vicenin-2) | 14,6±0,4 (lemon) |
| 5 | Chrysoeryol 6,8-di-*C*-Glu | 11,3±0,2 (lemon) |
| 6 | Eriocitrin | 83,3±5,6 (lemon) |
| 10 | Hesperidin | 133,7±15,9 (orange) |
| | | 6,3±0,1 (lemon) |
| 12 | Limocitrin HMG-Glu | 12,0±1,5 (lemon) |

| | | |
|---|---|---|
| Values are expressed as the mean ± standard deviation (SD) of an n=3 | | |

In the orange extract, hesperidin was only detected and quantified in the analyzed supernatant (after extraction with DMSO). No quantifiable amounts of the compound were observed in the orange extract precipitate.

Flavones were also detected in the lemon extract in significant quantities, such as apigenin 6,8-di-C-Glu (Vicenin-2) and chrysoeriol 6,8-di-C-Glu (Table 2). The flavonol, limocitrin HMG-Glu in an amount of 12 mg / g extract was also quantified. The flavones (compounds 2 and 5) were quantified using apigenin as a standard and the quantified flavonol, limocitrin HMG-Glu (compound 12), with rutin.

Example 4: Characterization of the metabolites after consuming lemon or orange extract.

Through a targeted metabolomic strategy, a total of 17 metabolites were tentatively identified (Table 3). No hesperidin or eriocitrin-derived metabolites were detected in any volunteer at baseline. All the identified metabolites were detected at least in one volunteer, independently of the extract consumed, except for the metabolites M9 (eriodictyol sulfoglucuronide) and M11 (hesperetin sulfoglucuronide), which only were detected after consuming lemon and orange extract, respectively (Table 3). The extracted ion chromatograms (EICs) of all the compounds detected are shown in Figure 3. These representative EICs illustrate the metabolites identified in urine (F3 fraction) after consuming lemon (Figure 3A) and orange (Figure 3B) extracts, and plasma after consuming lemon (Figure 3C) and orange (Figure 3D) extracts. From the 17 metabolites, 16 were detected after consuming lemon extract (except M11), and 15 after consuming orange extract (except M9 and M10 in urine, and M9 and M12 in plasma) (Table 3).

**Table 3. Plasma and urine metabolites identified in volunteers (n=16) after consuming lemon (LE) or orange (OE) extracts.**

| **Num** | **Metabolites** | **RT** | **m/z⁻** | **Occurrence**² | | | |
|---|---|---|---|---|---|---|---|
| | | | | Urine LE | Plasma LE | Urine LE | Plasma LE |
| **M1** | Eriodictyol glucuronide-1 | 6.37 | 463.0882 | 16 | 16 | 5 | 3 |
| **M2** | Eriodictyol glucuronide-2 | 7.31 | 463.0882 | 16 | 16 | 10 | 7 |
| **M3** | Naringenin 7-*O*-glucuronide¹ | 7.52 | 447.0933 | 16 | 16 | 16 | 14 |
| **M4** | Eriodictyol glucuronide-3 | 7.68 | 463.0882 | 16 | 16 | 11 | 7 |
| **M5** | Naringenin 4'-*O*-glucuronide¹ | 7.70 | 447.0933 | 16 | 16 | 16 | 14 |
| **M6** | Homoeriodictyol glucuronide | 7.72 | 477.1038 | 16 | 16 | 3 | 3 |
| **M7** | Hesperetin 7-*O*-glucuronide¹ | 8.02 | 477.1038 | 16 | 16 | 16 | 16 |
| **M8** | Hesperetin 3'-*O*-glucuronide¹ | 8.20 | 477.1038 | 16 | 16 | 16 | 16 |
| **M9** | Eriodictyol sulfoglucuronide | 8.99 | 543.0450 | 5 | 15 | 0 | 0 |
| **M10** | Homoeriodictyol sulfate | 9.02 | 381.0286 | 16 | 16 | 0 | 3 |
| **M11** | Hesperetin sulfoglucuronide | 9.30 | 557.0607 | 0 | 0 | 7 | 8 |
| **M12** | Eriodictyol¹ | 9.50 | 287.0561 | 14 | 16 | 4 | 0 |
| **M13** | Eriodictyol sulfate | 9.75 | 367.0129 | 16 | 16 | 12 | 10 |
| **M14** | Hesperetin 3'-*O*-sulfate¹ | 9.89 | 381.0286 | 16 | 16 | 16 | 16 |
| **M15** | Naringenin¹ | 11.22 | 271.0612 | 9 | 6 | 5 | 2 |
| **M16** | Homoeriodictyol¹ | 11.42 | 301.0718 | 12 | 15 | 1 | 1 |
| **M17** | Hesperetin¹ | 11.73 | 301.0718 | 5 | 8 | 8 | 3 |

| | | | | | | | |
|---|---|---|---|---|---|---|---|
| ¹The identification was achieved using authentic standards. Number of volunteers in whom each metabolite was detected after consuming lemon (LE) or orange (OE) extracts. | | | | | | | |

Table 4 and Table 5 show the urinary excretion of quantified metabolites at the different fractions collected, i.e., 0-4 h (F1), 4-8 h (F2), 8-10 h (F3), and 10-24 h (F4), after extracts intake. The total excretion (24 h) of metabolites was significantly higher after consuming lemon extract than after consuming orange extract, considering the total amount of flavanones consumed.

**Table 4. Metabolites quantified in urine fractions after consuming lemon extract.**

| **Lemon Extract** | | | | | | |
|---|---|---|---|---|---|---|
| **Num** | **Metabolites** | **F1** | **F2** | **F3** | **F4** | **Total F** |
| **M1** | Eriodictyol glucuronide-1 | 3.2 ± 5.9 | 9.6 ± 15.5 | 4.5 ± 6.2 | 0.7 ± 0.7 | 18.0 ± 28.4 |
| **M2** | Eriodictyol glucuronide-2 | 6.4 ± 11.0 | 18.0 ± 17.2 | 18.1 ± 19.7 | 4.2 ± 5.7 | 46.6 ± 53.6** |
| **M3** | Naringenin 7-*O*-glucuronide | 1.0 ± 0.7 | 2.6 ± 4.0 | 1.7 ± 1.9 | 0.3 ± 0.3 | 5.7 ± 6.9*** |
| **M4** | Eriodictyol glucuronide-3 | 9.4 ± 16.9 | 33.8 ± 64.1 | 27.7 ± 40.1 | 4.3 ± 6.4 | 75.1 ± 128* |
| **M5** | Naringenin 4'-*O*-glucuronide | 0.8 ± 0.5 | 1.7 ± 2.7 | 1.3 ± 1.5 | 0.4 ± 0.3 | 4.2 ± 5.0*** |
| **M6** | Homoeriodictyol glucuronide | 13.9 ± 16.2 | 48.1 ± 90.0 | 37.2 ± 56.9 | 6.8 ± 8.0 | 106 ± 171 |
| **M7** | Hesperetin 7-*O*-glucuronide | 1.2 ± 1.4 | 4.0 ± 4.7 | 2.7 ± 2.5 | 0.6 ± 0.6 | 8.5 ± 9.2*** |
| **M8** | Hesperetin 3'-*O*-glucuronide | 2.1 ± 2.3 | 10.7 ± 11.3 | 9.2 ± 7.7 | 2.2 ± 2.4 | 24.2 ± 23.7*** |
| **M10** | Homoeriodictyol sulfate | 3.8 ± 3.2 | 4.0 ± 5.5 | 3.6 ± 4.1 | 0.5 ± 0.4 | 9.6 ± 11.3 |
| **M13** | Eriodictyol sulfate | 4.6 ± 5.0 | 14.6 ± 17.3 | 11.9 ± 12.6 | 1.5 ± 1.5 | 32.6 ± 36.5*** |
| **M14** | Hesperetin 3'-*O*-sulfate | 1.9 ± 2.6 | 4.3 ± 4.5 | 3.3 ± 3.1 | 0.7 ± 0.6 | 9.0 ± 9.1* |
| **Total excretion (24h): 339 ± 482***** | | | | | | |

| | | | | | | |
|---|---|---|---|---|---|---|
| Values expressed as µg/mg creatinine (mean ± SD). F1: Fraction collected from 0 to 4 h after consuming lemon or orange extract; F2: Fraction collected from 4 to 8 h; F3: Fraction collected from 8 to 10 h; F4: Fraction collected at 24 h; Total F (24 h), sum of the fractions F1, F2, F3 and F4. **p* < 0.05; ***p* < 0.01; *** *p* < 0.001. | | | | | | |

**Table 5. Metabolites quantified in urine fractions after consuming orange extract.**

| **Orange Extract** | | | | | | |
|---|---|---|---|---|---|---|
| **Num** | **Metabolites** | **F1** | **F2** | **F3** | **F4** | **Total F** |
| **M1** | Eriodictyol glucuronide-1 | - | 0.4 ± 0.3 | 0.1 ± 0.1 | 0.05 ± 0.05 | 0.6 ± 0.4 |
| **M2** | Eriodictyol glucuronide-2 | 0.1 ± 0.1 | 0.5 ± 0.8 | 0.5 ± 0.6 | 1.4 ± 3.4 | 2.5 ± 4.9 |
| **M3** | Naringenin 7-*O*-glucuronide | 0.1 ± 0.1 | 0.2 ± 0.6 | 0.2 ± 0.2 | 0.2 ± 0.3 | 0.7 ± 1.5 |
| **M4** | Eriodictyol glucuronide-3 | 0.1 ± 0.2 | 0.5 ± 0.9 | 0.7 ± 0.7 | 2.1 ± 4.5 | 3.4 ± 6.2 |
| **M5** | Naringenin 4'-*O*-glucuronide | 0.1 ± 0.1 | 0.3 ± 0.5 | 0.3 ± 0.4 | 0.3 ± 0.5 | 1.0 ± 1.5 |
| **M6** | Homoeriodictyol glucuronide | - | | | | |
| **M7** | Hesperetin 7-*O*-glucuronide | 0.2 ± 0.6 | 2.1 ± 5.7 | 2.2 ± 3.6 | 1.1 ± 3.3 | 5.6 ± 13.2 |
| **M8** | Hesperetin 3'-*O*-glucuronide | 0.5 ± 1.5 | 4.3 ± 11.5 | 3.3 ± 4.2 | 1.8 ± 1.6 | 9.9 ± 18.9 |
| **M10** | Homoeriodictyol sulfate | - | | | | |
| **M13** | Eriodictyol sulfate | 0.02 ± 0.01 | 0.1 ± 0.1 | 0.2 ± 0.4 | 0.3 ± 0.4 | 0.6 ± 0.9 |
| **M14** | Hesperetin 3'-*O*-sulfate | 0.1 ± 0.3 | 1.2 ± 3.3 | 1.9 ± 3.1 | 0.6 ± 0.6 | 3.8 ± 7.3 |
| **Total excretion (24 h): 28 ± 55** | | | | | | |

| | | | | | | |
|---|---|---|---|---|---|---|
| Values expressed as µg/mg creatinine (mean ± SD). F1: Fraction collected from 0 to 4 h after consuming lemon or orange extract; F2: Fraction collected from 4 to 8 h; F3: Fraction collected from 8 to 10 h; F4: Fraction collected at 24 h; Total F (24 h), sum of the fractions F1, F2, F3 and F4. **p*< 0.05; ***p*< 0.01; ****p* < 0.001. | | | | | | |

## Claims

1. Powdered food composition, obtained from lemon **characterized in that** it comprises between 1% and 30% (w/w) of eriocitrin.

2. Powder composition according to claim 1, **characterized in that** the composition further comprises other flavonoids.

3. Powder composition according to claim 1 or 2, **characterized in that** said flavonoids are apigenin 6,8-di-C-Glu (Vicenin-2), chrysoeryiol 6,8-di-C-Glu and / or limocitrin HMG -Glu.

4. Powdered composition according to any of the preceding claims, **characterized in that** the eriocitrin is extracted from a lemon extract.

5. Powder composition according to any of the preceding claims, **characterized in that** the eriocitrin is extracted from the lemon fruit.

6. Powder composition according to claim 5, **characterized in that** it is obtained from the fruit of Citrus limon (L.) Osbeck.

7. Method for obtaining a powder composition according to any of the preceding claims, **characterized in that** the eriocitrin is obtained by a process comprising a solvent extraction step and a tangential flow filtration step.

8. Method according to claim 7, **characterized in that** the size of the membrane in tangential flow filtration is between 100 Da and 100,000 Da.

9. Powdered food composition according to claims 1 to 6, for use in the treatment of a disease selected from the group consisting of inflammation, cancer, neurodegenerative diseases, diabetes, cardiovascular diseases, vascular diseases and cutaneous diseases **characterized in that** said composition is administered in a dose of eriocitrin between 5 and 35 mg per day.

10. Powdered food composition according to claim 9, **characterized in that** said composition is administered in a dose of eriocitrin between 7 and 30 mg per day.
